# EUROPÄISCHE PATENTSCHRIFT

(11) **EP 3 258 111 B1**
(45) Veröffentlichungstag und Bekanntmachung des Hinweises auf die Patenterteilung: **15.08.2018**
(21) Anmeldenummer: 16174475.0
(22) Anmeldetag: 14.06.2016
(51) Int. Cl.: F04B 1/02, F04B 49/06, A61B 17/00, F04B 53/00, F04B 53/14, F04B 53/22, A61B 17/3203

(54) **PUMPMODUL**
PUMP MODULE
MODULE DE POMPE

(43) Veröffentlichungstag der Anmeldung: 20.12.2017
(73) Patentinhaber: MEDAXIS AG, 6340 Baar (CH)
(72) Erfinder: WIDMER, Beat, 6005 Luzern (CH)
(74) Vertreter: Grünecker Patent- und Rechtsanwälte PartG mbB

(56) Entgegenhaltungen:
- DE-A1-102004 031 673
- DE-A1-102014 016 141
- US-A- 3 922 115

## Beschreibung

Die vorliegende Erfindung betrifft ein Pumpmodul mit den oberbegrifflichen Merkmalen von Anspruch 1. Ein solches Pumpmodul ist aus der DE 10 2014 016 141 A1 bekannt.

Die vorliegende Erfindung will insbesondere ein Pumpmodul angeben, welches sich kostengünstig herstellen lässt.

Dabei will die vorliegende Erfindung insbesondere ein Pumpmodul für medizinische Zwecke angeben, speziell für das Ventil und mittels Wasserstrahl. Bei dieser Vorgehensweise wird ein konzentrierter Wasserstrahl auf eine Wunde aufgestrahlt, um zur Förderung der Wundheilung Schorf abzutragen.

Da bei diesem Vorgehen vermieden werden muss, dass im Rahmen der Behandlung erneut Keime und Verunreinigungen in die Wunde eingebracht werden, muss das entsprechende Pumpmodul sterilisierbar sein bzw. bereits sterilisiert in Verkehr gebracht und sterilisiert verpackt sein, bevor das Pumpmodul im Rahmen der Therapie zum Einsatz kommt. Dies wiederum bedingt die Herstellung des Pumpmoduls unter sterilen Bedingungen, was aufwändig ist.

Ebenso aufwendig ist es, das Pumpmodul vor der Benutzung in seine wesentlichen Bestandteile zu zerlegen und zu sterilisieren. Denn das erfindungsgemäße Pumpmodul ist üblicherweise ein Einwegartikel, der üblicherweise nur einmal verwendet wird.

Die vorliegende Erfindung betrifft insbesondere ein Pumpmodul mit einem Gehäuse, in dem zumindest ein Pumpkolben reversierend beweglich gelagert ist, der mit zumindest einem Dichtelement versehen ist, das in einem Pumpbetrieb dichtend an einem Zylinder anliegt. Ein solches Pumpmodul für den Einsatz in der Chirurgie ist beispielsweise aus der US 2010/0049228 bekannt. Dieses Pumpmodul hat zu jedem Pumpkolben einen Faltenbalg, der sich zwischen dem Gehäuse und dem Pumpkolben erstreckt, um den mit dem Zylinder zusammenwirkenden Bereich des Pumpkolbens gegenüber der Umgebung abzudichten und so den Eintrag von Bakterien zu verhindern.

Solche Pumpmodule der eingangs genannten Art sind beispielsweise aus der US 2002/0176788 A1, der US 2014/0079580 A1, der EP 1 768 580 A1 oder der US 2011/0150680 A1 bekannt. Mitunter werden diese vorbekannten Pumpmodule als sterilisierbar bezeichnet.

Es gibt verschiedene Möglichkeiten, medizinisches Gerät zu sterilisieren bzw. zu desinfizieren. Notwendig für die wirksame Vorbereitung von chirurgischem Gerät ist indes der Kontakt zwischen dem Desinfektions- bzw. Sterilisationsmittel und den zu sterilisierenden Teilen. Bei Pumpmodulen der eingangs genannten Art liegt im Pumpbetrieb das Dichtungselement an dem
Pumpkolben dichtend an, was es schwierig bis unmöglich macht, in diesem Bereich eine wirkungsvolle Sterilisation bzw. Desinfektion zu erreichen. Aber gerade dort wird das auf den Körper aufzustrahlende Fluid gepumpt. So besteht die Notwendigkeit, für eine wirkungsvolle Vorbereitung des Pumpmodules den Pumpkolben aus dem Gehäuse zu entnehmen, zu sterilisieren und nach der Sterilisation die Bauteile zusammenzusetzen.

Die vorliegende Erfindung will ein Pumpmodul der eingangs genannten Art angeben, welches sich kostengünstig herstellen und einfach sterilisieren bzw. desinfizieren lässt.

Zur Lösung dieser Aufgabe wird mit der vorliegenden Erfindung ein Pumpmodul gemäß Anspruch 1 vorgeschlagen. Bei diesem Pumpmodul ist der Pumpkolben über ein an dem Gehäuse angeordnetes Sicherungselement, das in der Parkposition mit einem an dem Pumpkolben vorgesehenen Sicherungsgegenelement zur Festlegung des Pumpkolbens in der Parkposition zusammenwirkt, festlegbar. Als Zylinder im Sinne der vorliegenden Erfindung wird dabei derjenige Bereich angesehen, an den das Dichtelement dichtend anliegt. Das Dichtelement hat daher Kontakt mit der zylindrischen Innenumfangsfläche des Zylinders. In der Parkposition befindet sich das Dichtelement außerhalb dieses Bereiches des Zylinders mit zylindrischer Innenumfangsfläche.

Das erfindungsgemäße Pumpmodul hat üblicherweise einen Antriebsbereich, zu dem der Zylinder offen ist, und an dem der Pumpkolben mit seinem antriebsseitigen Ende freiliegt, um mit einem Antrieb verbunden zu werden. Das den Antrieb aufnehmende Gestellt hat dabei üblicherweise eine Aufnahme für das Pumpmodul, so dass dieses fixiert und fest mit dem Antrieb verbunden werden kann. Auf der gegenüberliegenden Seite des Antriebsbereiches ist üblicherweise ein Ausgabebereich vorgesehen, an dem sich eine Ausgabeöffnung für das unter Druck gesetzte Fluid befindet. Unter Zugrundelegung dieser konstruktiven Ausgestaltung befindet sich üblicherweise das Dichtelement in der Parkposition dem Zylinder vorgelagert, d. h. in Richtung auf den Antriebsbereich vorgelagert, so dass der Pumpkolben für den Pumpbetrieb zunächst von der Parkposition in Richtung auf den Ausgabebereich vorgeschoben werden muss, um das Dichtelement dichtend an den Zylinder anzulegen. Diese Bewegung entspricht üblicherweise der Fügebewegung des Pumpmoduls zur Kopplung des Pumpkolbens mit dem Antrieb, so dass bei der hier diskutierten Weiterbildung auf einfache Weise der Pumpkolben von der Parkposition in eine Pumpposition in Pumpbetrieb verbracht wird, so dass das Pumpmodul nach Koppeln mit dem Antrieb zwangsläufig einen im Pumpbetrieb vorgesehenen Pumpkolben hat. Das Anschalten des Antriebs erlaubt daher den unmittelbaren Betrieb des Pumpmoduls zum Ausgeben eines Flüssigkeitsstrahls.

Bei dem erfindungsgemäßen Pumpmodul ist eine Festlegung des Pumpkolbens in der Parkposition verwirklicht. Als Festlegemittel ist jedes beliebige Mittel denkbar, welches einer Verschiebebewegung des Pumpkolbens relativ zu dem Gehäuse einen gewissen Widerstand entgegensetzt, jedoch nicht verhindert, dass bei Überschreiten einer vorbestimmten axialen Kraft der Pumpkolben axial in eine Betriebsposition verschoben werden kann, in welcher das Dichtelement dichtend an der Innenumfangsfläche des Zylinders anliegt, so dass der reversierende Betrieb des Pumpkolbens Fluid innerhalb des Pumpmodul unter Druck setzt und ausfördert. Als Festlegemittel kann ein externes oder gesondertes Sicherungselement vorgesehen sein, welches den Pumpkolben relativ zu dem Gehäuse festlegt, und welches vor dem Betrieb entfernt werden muss, um den Pumpkolben innerhalb des Gehäuses reversierend beweglich zu machen. Ein solches Festlegemittel kann beispielsweise ein Sprengring sein, der den Pumpkolben außenumfänglich umgibt, mit diesem formschlüssig verbunden ist und mit einer Gegenfläche, die von dem Gehäuse gebildet wird, zusammenwirkt oder daran anliegt, um das Vortreiben des Pumpkolbens in eine der Pumppositionen zu verhindern und die Parkposition festzulegen.

Solche Ausgestaltungen sind zwar durchaus denkbar. Sie haben aber den Nachteil, dass das Aufheben der Parkposition aufwändig ist. Gemäß einer bevorzugten Weiterbildung der vorliegenden Erfindung ist das Sicherungselement einteilig an dem Gehäuse angeformt. Denkbar ist, das Gehäuse und damit auch das Sicherungselement aus Kunststoff herzustellen. Das Sicherungselement kann beispielsweise eine Rastklinke sein, die einteilig an dem Gehäuse ausgebildet ist und elastisch radial gegen den Pumpkolben vorgespannt ist, um mit diesem in der Parkposition in Wechselwirkung zu treten und diesen in der Parkposition festzulegen. Dabei kann das Sicherungsmittel reibschlüssig und/oder formschlüssig mit dem Pumpkolben zusammenwirken.

Gemäß einer bevorzugten Weiterbildung der vorliegenden Erfindung wird indes eine formschlüssige Verbindung vorgeschlagen, bei welcher das Sicherungselement bzw. das Sicherungsgegenelement zwei mit axialen Abstand zueinander vorgesehene radiale Vorsprünge umfasst, zwischen denen das andere von Sicherungselement und Sicherungsgegenelement in der Parkposition eingreift. Durch die Radialvorsprünge wird dementsprechend eine Nut ausgebildet die sich zum formschlüssigen Eingriff eignet. Die Radialvorsprünge können dabei durch Ausformen einer Nut an einer zylindrischen Außenumfangsfläche des Pumpkolbens ausgeformt sein. In diesem Fall erheben sich die Radialvorsprünge lediglich von dem Grund der Nut radial nach außen bis zu der glatten Außenumfangsfläche des Kolbens ab. Die Radialvorsprünge können aber ebenso gut den im Wesentlichen zylindrischen Pumpkolben radial nach außen überragen, wodurch definierte Führungsflächen gebildet werden können, die im Pumpbetrieb an einer Zylinderwand des Gehäuses geführt sein können, welche dem Zylinder axial vorgelagert ist. Diese Zylinderwand wird üblicher weise durch einen durch das Gehäuse gebildeten Führungszylinder ausgeformt Dadurch kann verhindert werden, dass der Pumpkolben während des Betriebs knickt. Auch wird eine gute axiale Ausrichtung des Pumpkolbens durch Führung einerseits über das Dichtelement und andererseits über das Sicherungsgegenelement ermöglicht.

Gemäß einer bevorzugten Weiterbildung der vorliegenden Erfindung ist an dem Zylinder ein konischer Einzug vorgelagert vorgesehen. Dieser konische Einzug ist bevorzugt unmittelbar dem Zylinder vorgelagert vorgesehen. Der Einzug hat einen kleinen Innendurchmesser, der im Wesentlichen dem Innendurchmesser des Zylinders entspricht. Durch diesen konischen Einzug kann die Montage des Pumpkolbens zusammen mit dem Dichtelement vereinfacht werden. Der konische Einzug ist insbesondere so ausgebildet, dass er das Dichtelement im Wesentlichen auf einen Außendurchmesser bringt, der dem Innendurchmesser des Zylinders entspricht, wobei das Dichtelement konzentrisch zu dem Zylinder angeordnet wird, so dass das Einbringen des Dichtelementes in den Zylinder vereinfacht ist. Dabei kann der konische Einzug an dem Gehäuse vorgesehen sein.

Mit Blick auf eine möglichst effektive Nutzung der reversierenden Bewegung des Antriebes zum Pumpen wird gemäß einer bevorzugten Ausgestaltung der vorliegenden Erfindung vorgeschlagen, das Dichtelement in der Parkposition in dem konischen Einzug vorzusehen. Eine geringfügige axiale Verschiebung des Pumpkolbens nach dem Verbinden des Pumpmodules mit dem Antrieb führt dementsprechend unmittelbar dazu, dass sich das Dichtelement innerhalb des Zylinders und damit in einer Pumpposition befindet. Dies gilt insbesondere dann, wenn der konische Einzug am Eingang des Zylinders ausgebildet ist und absatzfrei in die Zylinderfläche übergeht..

Gemäß einer bevorzugten Weiterbildung der vorliegenden Erfindung kann der konische Einzug durch einen Zylindereinsatz gebildet sein, an dem das Dichtelement im Pumpbetrieb dichtend anliegt. Diese Ausgestaltung bietet den Vorteil, dass das Gehäuse ganz überwiegend aus einem kostengünstigen Kunststoff hergestellt werden kann, die mit dem Dichtelement in Wechselwirkung tretende Dichtfläche des Zylinders in das durch einen Zylindereinsatz, der beispielsweise aus einem technisch hochwertigen Kunststoff oder aus Metall hergestellt und dichtend in das Gehäuse eingesetzt werden kann. Als Kunstoffe zur Herstellung des Pumpmoduls oder Teile davon kommen PA, PE, PP und/oder POM in Frage. So nutzt die Weiterbildung einer kostengünstigen Herstellung des Pumpmodules bei guter Präzision und Abstimmung zwischen dem Dichtelement und der isolierende Ummantelungsfläche des Zylinders, welche bevorzugt exakt mit ihrem Durchmesser auf den Außendurchmesser des Dichtelementes abgestimmt und mit guter Oberflächenqualität und glatt ausgebildet sein sollte.

Gemäß einer weiteren bevorzugten Ausgestaltung der vorliegenden Erfindung umfasst der Pumpkolben einen Stößelkörper, der an einem Ende ein Formschlusselement zur Koppelung des Kolbens mit den dem Kolben zugeordneten Antrieb aufweist und an seinem anderen Ende mit einer Aufnahme für das Dichtelement versehen ist. Dieser Stößelkörper formt dabei bevorzugt den gesamten Pumpkolben aus, wobei der Stößelkörper für sich üblicherweise Befestigungsmittel, wie beispielsweise Formschlussmittel ausbildet, die das Dichtelement an dem Stößelkörper halten. So kann der Stößelkörper beispielsweise aus Kunststoff und damit kostengünstig hergestellt sein.

Bei der zuvor diskutierten Weiterbildung überragt das Formschlusselement bevorzugt in der Parkposition das Gehäuse, so dass ein optischer Indikator für den Benutzer der Pumpe gegeben ist, um festzustellen, ob der Pumpkolben sich vor und nach der Desinfektion bzw. Sterilisation in der Parkposition befunden hat. Ebenso gut kann der Pumpkolben mit einem anderen optischen Indikator, beispielsweise einer farblichen oder konturierten Gestaltung versehen sein, deren Position überprüfbar ist, beispielsweise durch ein an dem Gehäuse ausgebildetes Sichtfenster, um zu überprüfen, ob sich der Pumpkolben tatsächlich in der Parkposition befindet und somit eine den Anforderungen genügende Vorbereitung des Pumpmodules vor der Benutzung durch Desinfizieren bzw. Sterilisieren erfolgt ist. Das Desinfizieren kann beispielsweise durch Einleiten eines Ethylenoxid-Gases in das Pumpmodul erfolgen (Ethylenoxid (EO) Sterilisation). Dieses Gas umströmt auch den in der Parkposition befindlichen Pumpkolben und auch das Dichtelement, so dass dieses vollständig von der Desinfektion erfasst wird.

Der in der Parkposition vorgesehene Spalt zwischen dem Außenumfang des Dichtelementes und dem Innenumfang der das Dichtelement umgebenden Wandungen des Gehäuses beträgt zumindest 1/10 mm, bevorzugt zumindest 2/10 mm und besonders bevorzugt zumindest 3/10 mm. Im Hinblick auf eine zügige Überführung des Pumpkolbens von der Parkposition in eine Betriebs- bzw. Pumpposition, in welcher das Dichtelement dichtend an dem Zylinder anliegt, befindet sich das Dichtelement in der Parkposition bevorzugt nahe zu dem Zylinder. Der zuvor erwähnte Spalt kann dabei durch den konischen Einzug oder einen anderweitig, üblicherweise durch das Gehäuse ausgebildeten Bereich geformt sein, der dem Zylinder bevorzugt unmittelbar vorgelagert ist. Wesentlich für die Einstellung des Spaltmaßes ist ein freier Durchgang des Desinfektionsmittels, so dass dieses sämtliche Oberflächen des Dichtelementes bedecken kann. Als Dichtelement i. S. d. vorliegenden Erfindung wird dabei jedes Element verstanden, welches in der Lage ist, abdichtend gegen die Innenumfangswand des Zylinders angelegt zu werden, um einen Pumpbetrieb zu ermöglichen. Dabei kann das Dichtelement auch einteilig an dem Pumpkolben ausgeformt sein.

Gemäß einer bevorzugten Weiterbildung der vorliegenden Erfindung wird das eine Ende in der Parkposition von dem Gehäuse überragt und/oder ist von einer lösbar mit dem Gehäuse verbundenen Abdeckkappe überdeckt. Diese Weiterbildung soll verhindern, dass der Pumpkolben vor dem Einbau in eine Antriebseinrichtung und vor der Desinfektion versehentlich von der Parkposition in eine Betriebsposition verschoben wird, in welcher das Dichtelement dichtend an den Zylinder angelegt wird.

Das Gehäuse hat bevorzugt eine Formgestaltung, die es ermöglicht, das Gehäuse in einer Bewegung, die im Wesentlichen der axialen Bewegung des reversierend beweglichen Pumpkolbens entspricht, mit dem Antrieb verbunden zu werden. Hierzu hat das Gehäuse sich üblicherweise parallel zu der Bewegungsrichtung des Pumpkolbens erstreckende Führungsflächen, welche das Einbringen des Pumpmodules in ein Gestell oder Gehäuse des Antriebs ermöglichen, um den Pumpkolben mit seinem antriebsseitigen Ende gegen den Antrieb anzulegen und dabei bevorzugt im Rahmen des Fügens von Pumpmodul und Antrieb den Pumpkolben mit dem Antrieb so zu koppeln, dass der Pumpkolben reversierend beim Betrieb des Antriebes bewegt wird. Bei dieser Fügebewegung wird bevorzugt der Kolben von der Parkposition in eine Pumpposition verschoben. Im Rahmen des Einbaus des Pumpmoduls in das Gehäuse bzw. Gestell des Antriebs wird bevorzugt eine formschlüssige Verbindung zwischen dem Formschlusselement des Pumpkolbens und dem Antrieb im Rahmen des Einbaus des Pumpmoduls in das Gehäuse bzw. Gestell des Antriebs bewirkt.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
Fig. 1 eine erste Explosionsdarstellung des Ausführungsbeispiels;
Fig. 2 eine Explosionsdarstellung gemäß Fig. 1 für eine Pumpeinheit des in Fig. 1 gezeigten Ausführungsbeispiels;
Fig. 3 eine perspektivische Seitenansicht gemäß der Explosionsdarstellung nach Fig. 1 des montierten Pumpmoduls auf den vorderen Ausgabebereich;
Fig. 4 eine perspektivische Seitenansicht gemäß Fig. 3 in den offenen Antriebsbereich;
Fig. 5 eine Schnittdarstellung entlang der Linie V-V gemäß Fig. 4 bzw. Fig. 11, wobei die Schnittebene die Mittellängsachse des Pumpenmoduls enthält;
Fig. 6 eine Schnittansicht entlang der Linie V-V gemäß Fig. 4, wobei die Schnittlinie die Bewegungsebene des Pumpkolbens enthält;
Fig. 7 das vergrößerte Detail VII gemäß Fig. 6;
Fig. 8 das vergrößerte Detail VIII gemäß Fig. 6 bei einem gegenüber Fig. 6 tiefer in den Zylinder eindringenden Pumpkolben;
Fig. 9 eine Schnittdarstellung entlang der Linie IX-IX gemäß Fig. 4, wobei die Schnittebene die Bewegungsebene und die Mittelängsachse eines der Pumpkolben enthält;
Fig. 10 das vergrößerte Detail X gemäß Fig. 9;
Fig. 11 eine perspektivische rückseitige Ansicht ähnlich Fig. 4;
Fig. 12 das vergrößerte Detail XII gemäß Fig. 11;
Fig. 13 eine perspektivische rückseitige Ansicht der Pumpeinheit nach Fig. 2;
Fig. 14 eine Schnittdarstellung entlang der Linie XIV-XIV gemäß der Darstellung in Fig. 13;
Fig. 15 das Detail XV gemäß Fig. 14;
Fig. 16 eine rückseitige Draufsicht auf das Ausführungsbeispiel;
Fig. 17 eine Schnittdarstellung entlang der Linie XVII-XVII gemäß der Darstellung nach Fig. 16, wobei die Schnittebene die Mittellängsachsen der beiden Spannschrauben 6 enthält und parallel zu der Bewegungsebene des Pumpkolbens verläuft;
Figur 18 eine perspektivische Ansicht eines Ausführungsbeispiels einer Vorrichtung zur Erzeugung eines Fluidstrahles;
Figur 19 das Detail nach Figur 18 in vergrößerter Darstellung ohne Pumpenmodul;
Figur 20 das Detail nach Figur 18 in einer Draufsicht;
die Figuren 21a-c eine Ansicht ähnlich zu Fig. 19 mit einer Abfolge von Schritten beim Fügen des Pumpenmoduls und
Figur 22a-c teilweise geschnittene Draufsichten auf die zusammenwirkenden Enden von Antriebselement und Antriebsgegenelement und deren relative Lage beim Schwenken im Rahmen des Fügens; .

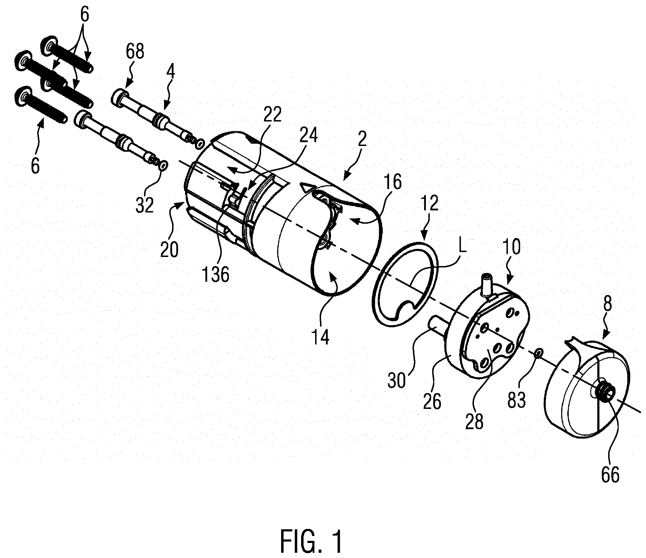

Die Fig. 1 zeigt die wesentlichen Bestandteile des Ausführungsbeispiels nach der vorliegenden Erfindung, bei dem es sich um ein Pumpmodul handelt. Das Pumpmodul hat eine Gehäusebasis 2, die zwei Stößelkörper 4 in sich aufnimmt und reversierend beweglich umgibt. Weiterhin sind vier Spannschrauben 6 dargestellt, die Ausführungsführungsbeispiele von Spannelementen im Sinne der vorliegenden Erfindung sind und im montierten Zustand im Eingriff mit einem Kopfelement 8, welches einer Pumpeinheit 10 vorgelagert ist, die unter Zwischenlage eines ringförmigen RFID-Elementes 12, der ein Beispiel einer Transpondereinheit der vorliegenden Erfindung darstellt, in der Gehäusebasis 2 aufgenommen ist. Hierzu hat die Gehäusebasis 2 einen Ausgabebereich 14, der als zylinderförmige Ausnehmung an der Gehäusebasis 2 ausgebildet ist, wobei ein Axialschlitz 16 zur Aufnahme eines Einlassanschlussstutzens 18 der Pumpeinheit 10 angepasst ausgebildet ist. An dem dem Ausgabebereich 14 gegenüberliegenden Ende ist die Gehäusebasis 2 ebenfalls offen und bildet einen Antriebsbereich 20 aus.

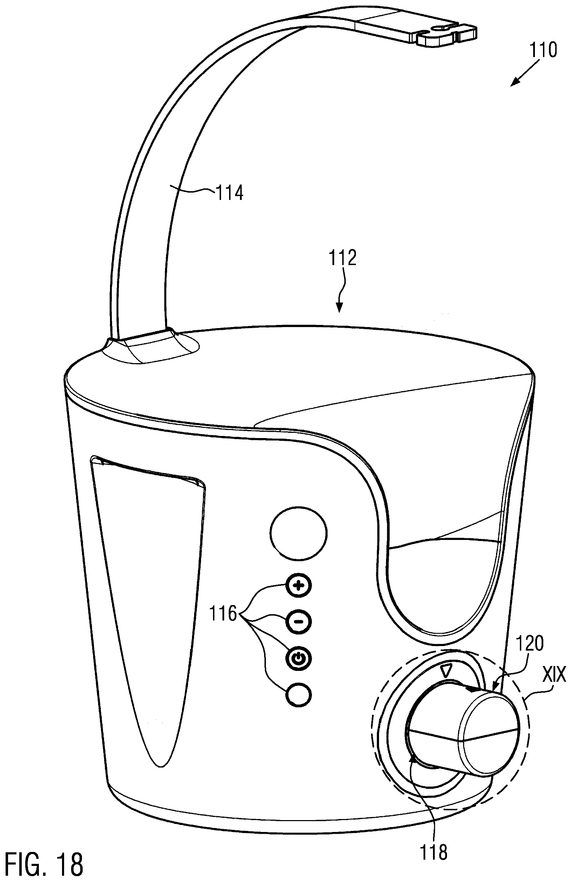

Wie Fig. 1 erkennen lässt, ist die Gehäusebasis im Wesentlichen zylindrisch ausgebildet. An der Außenumfangsfläche sind an der Gehäusebasis 2 eine sich in axialer Richtung der Gehäusebasis 2 erstreckende Nuten 22 und jeweils davon abgehende, sich quer hierzu erstreckende Quernuten 24 ausgebildet, die Führungs- und Verriegelungsflächen zur Befestigung des Pumpmoduls an einem Antriebsgehäuse darstellen, dessen Details in den Fig. 18 ff. und der zugehörigen Beschreibung erläutert sind.

Die Pumpeinheit 10 wird durch einen Ventilblock 26 und ein daran anliegendes Abdeckelement 28 gebildet, wobei von dem Ventilblock 26 auf der dem Abdeckelement 28 gegenüberliegenden Seite zwei Zylindereinsätze 30 abragen, von denen in Fig. 1 lediglich der eine Zylindereinsatz 30 zu erkennen ist, und die im Pumpbetrieb mit den Stößelkörpern 4 zusammenwirken. Die Stößelkörper 4 tragen hierzu jeweils ein Dichtelement 32 in Form eines Dichtringes, der formschlüssig im Bereich des vorderen freien Endes des Stößelkörpers 4 an diesem gehalten ist.

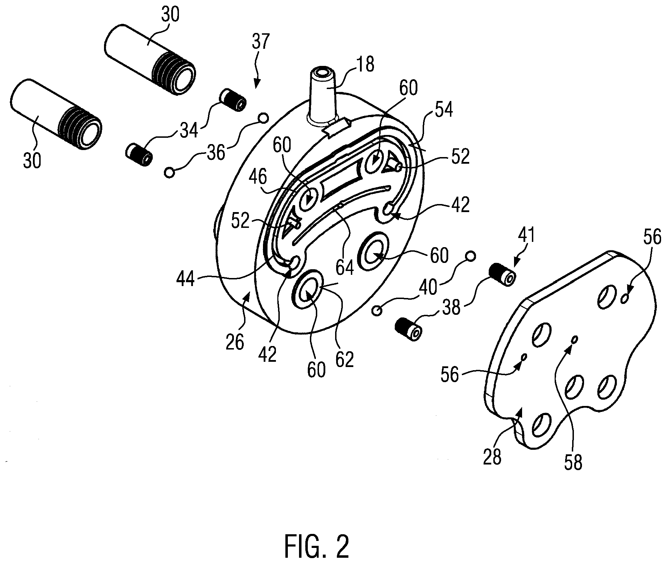

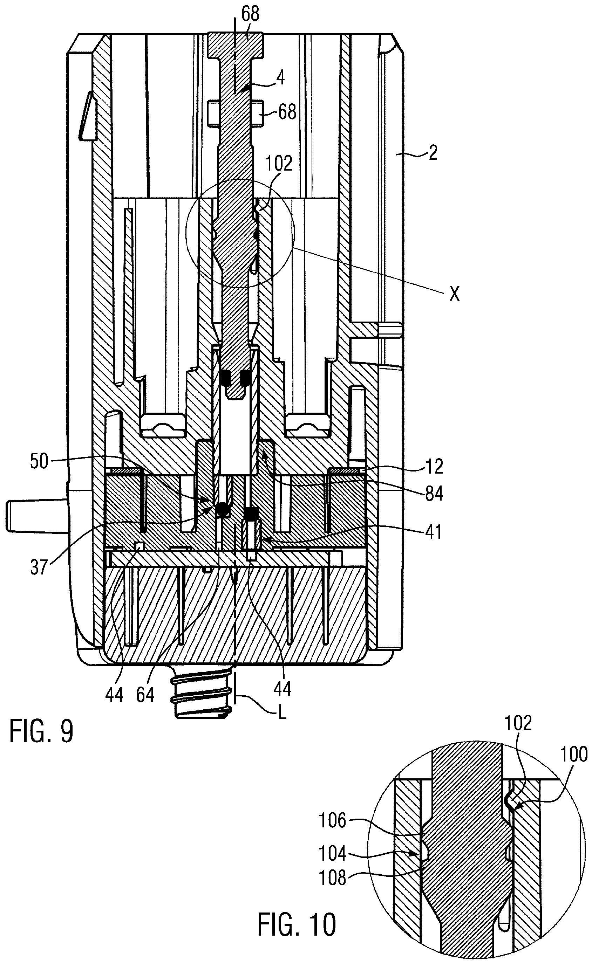

Fig. 2 lässt erkennen, dass die beiden Zylindereinsätze 30 an ihrem dem Ventilblock 26 zugewandten Ende an ihrem Außenumfang eine wellenförmige Konturierung aufweisen, die zum dichtenden Einsetzen der Zylindereinsätze 30 in den Ventilblock 26 ausgeformt sind. Zwischen den Zylindereinsätzen 30 und dem Ventilblock 26 ist jeweils eine Ventilbuchse 34 vorgesehen, die zusammen mit einer Ventilkugel 36 jeweils ein Auslassventil 37 bildet. Auf der den Zylindereinsätzen 30 gegenüberliegenden Seite sind Ventilbuchsen 38 mit zugehörigen Ventilkugeln 40 dargestellt, die zu den jeweiligen Zylindereinsätzen 30 Einlassventile 41 ausformen. Die Einlassventile 41 sind in Einlassventilbohrungen 42 aufgenommen, die in dem Ventilblock 26 ausgespart sind und mit einem Einlasskanal 44 kommunizieren, der in einem Vorsprung 46 als einseitig offene U-förmige Nut ausgespart ist und über das Abdeckelement 28 abgedeckt ist. Die Auslassventile 37 sitzen in entsprechenden Auslassventilbohrungen, von der einer in Fig. 9 beispielhaft gezeigt und mit Bezugszeichen 50 versehen ist. Wie Fig. 2 verdeutlicht, ist der Einlassanschlussstutzen 18 einteilig an dem Ventilblock 26 ausgebildet. Von der im Abdeckelement 28 zugewandten Seite ragen zwei Passelemente 52 mit unterschiedlichem Durchmesser von der durch den Vorsprung 46 gebildeten Dichtfläche 54 ab und überragen diese. Das Abdeckelement 28 hat für diese Passelemente 52 angepasst ausgebildete Bohrungen 56, die der richtigen Positionierung des Abdeckelementes 28 relativ zu dem Ventilblock 26 dienen. Dabei haben die Passelemente 52 und die Passbohrungen 56 einander jeweils angepasste Durchmesser, sodass nach einer Poka-Yoke-Funktion das Abdeckelement 28 immer in der richtigen Ausrichtung und Positionierung bei der Montage des Zylindereinsatzes 30 an dem Ventilblock 26 angeordnet wird.

Neben diesen beiden Passbohrungen 56 weist das Abdeckelement 28 noch eine Auslassbohrung 58 auf.

Der Ventilblock 26 hat korrespondierend zu den Spannschrauben 6 vier Durchgangsbohrungen 60, die einerseits die durch den Vorsprung 46 gebildete Dichtfläche 54 und andererseits Ringflächen 62 durchsetzen, die zur Anlage an das Abdeckelement 28 angepasst ausgebildet und höhengleich vorgesehen sind. An den Flächen 62 und 54 liegt das Abdeckelement 28 dichtend an und ist dagegen mittels Laser-Durchstrahl-Schweißen verschweißt. Hierzu ist das Abdeckelement 28 aus einem Laser-transparenten Material ausgebildet, wohingegen der Ventilblock 46 aus einem Laserstrahlen absorbierenden Kunststoffmaterial ausgeformt ist. Beide Teile können dementsprechend mittels Laser-Durchstrahl-Schweißen verbunden werden, wobei das aus Kunststoff ausgebildete Abdeckelement 28 an der Phasengrenze zu dem Ventilblock 26 mit dem Kunststoffmaterial des Ventilblocks 26 stoffschlüssig verbunden wird. Dadurch werden die Einlasskanäle 44 und ein mit Bezugszeichen 64 gekennzeichnete Auslasskanal, der eine an dem Ventilblock 26 ausgesparte U-förmige Rinne umfasst, die das Abdeckelement 28 abgedeckt ist, gebildet. Der Auslasskanal 64 kommuniziert über die Auslassbohrung 58 mit einer an dem Kopfelement 8 einteilig ausgebildeten Auslassanschlusstülle 66, die in axialer Verlängerung der Auslassbohrung 58 vorgesehen und an ihrem Außenumfang zur Ausbildung eines Luer-Anschlusses mit einem Außengewinde versehen ist. Über eine Überwurfmutter kann dementsprechend auf einfache Weise ein Druckschlauch über eine Luer-Verbindung an die Auslassanschlusstülle 66 angeschlossen werden.

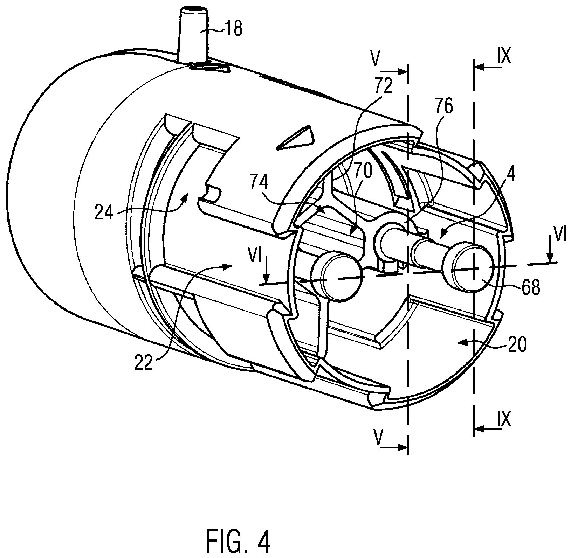

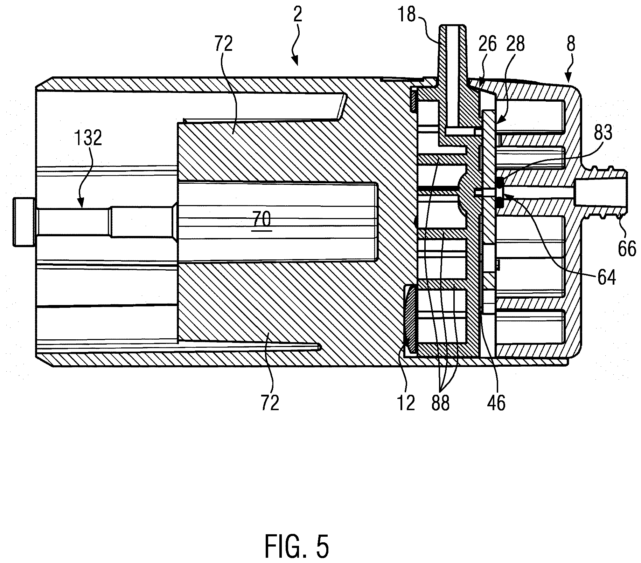

Die Fig. 4 zeigt eine perspektivische Seitenansicht mit einer Draufsicht auf das stirnseitige Ende der Gehäusebasis 2 und des Antriebsbereiches 20. Dabei sind die Stößelkörper 4 von der Gehäusebasis 2 umgeben und ragen mit ihrem einen Ende in den Antriebsbereich 20 hinein. Wie insbesondere Fig. 5 verdeutlicht, überragt das antriebsseitige Ende des Stößelkörpers 4, welches ein als Hammerkopf 68 ausgebildetes Formschlusselement ausbildet, das die Gehäusebasis 2 endseitig überragt. Ansonsten ist der Stößelkörper 4 aber axial von der Gehäusebasis überdeckt (vgl. Figur 5).

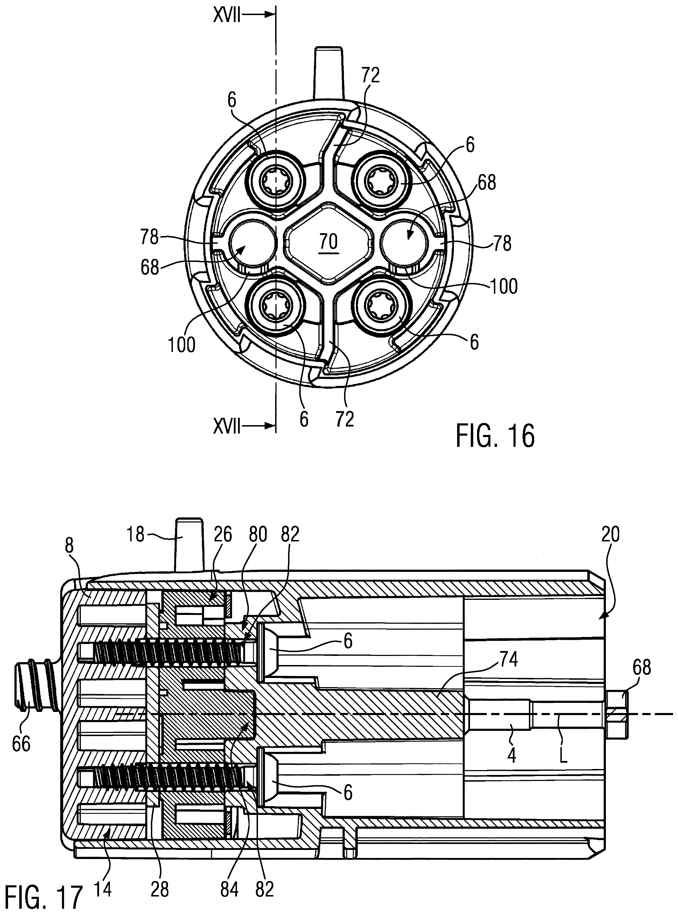

Wie die Zusammenschau der Fig. 4, 5, 6 und 11 verdeutlicht, ist die Gehäusebasis 2 als Spritzgussteil mit relativ gleichmäßigen Wandstärken ausgebildet, sodass sich bei der spritzgießtechnischen Herstellung der Gehäusebasis 2 ein gutes Erstarrungsverhalten ergibt. Als Kunststoffe zur Herstellung der Bauteile des Moduls kommen PA, PE, PP und/oder POM gegebenenfalls als gefüllte Kunststoffe, z. B. gefüllt mit Mineralien und/oder Fasern, in Frage. Dazu hat die Gehäusebasis 2 eine mittlere Ausnehmung 70, die über radiale Stege 72 an die Außenumfangsfläche der Gehäusebasis 2 angebunden ist, wobei die radialen Stege 72 von einer Polygonstruktur 74 abgehen, die zwischen den radialen Stegen 72 Führungsbuchsen 76 zu den jeweiligen Stößelkörpern 4 nach innen anbindet, die über weitere Radialstege 78 an der Außenumfangsfläche der Gehäusebasis 2 abgestützt sind (vgl. Fig. 16).

Die Gehäusebasis 2 bildet eine sich radial erstreckende Trennwand 80 aus, die unter anderem mit Durchtrittsbohrungen 82 für die Spannschrauben 6 versehen ist (vgl. Fig. 17). Dabei durchsetzen die Spannschrauben 6 die Trennwand 80, den Ventilblock 26 und das Abdeckelement 28 vollständig und das Kopfelement 8 teilweise und sind in diesem in Gewindeeingriff. Hierzu sind die Spannschrauben 6 selbstschneidend. Ebenso gut kann das Kopfelement 8 durch Verschweißen mit der durch die Gehäusebasis 2 in dem Ausgabebereich 14 gebildeten Ausnehmung verschweißt und damit mittelbar mit dem Ventilblock 26 und dem Abdeckelement 28 verbunden sein. Ein Dichtring 83 dichtet den durch die Auslassanschlusstülle 66 gebildeten Kanal gegenüber der Auslassbohrung 58 des Abdeckelementes 28 ab (vgl. Fig. 1 und 5).

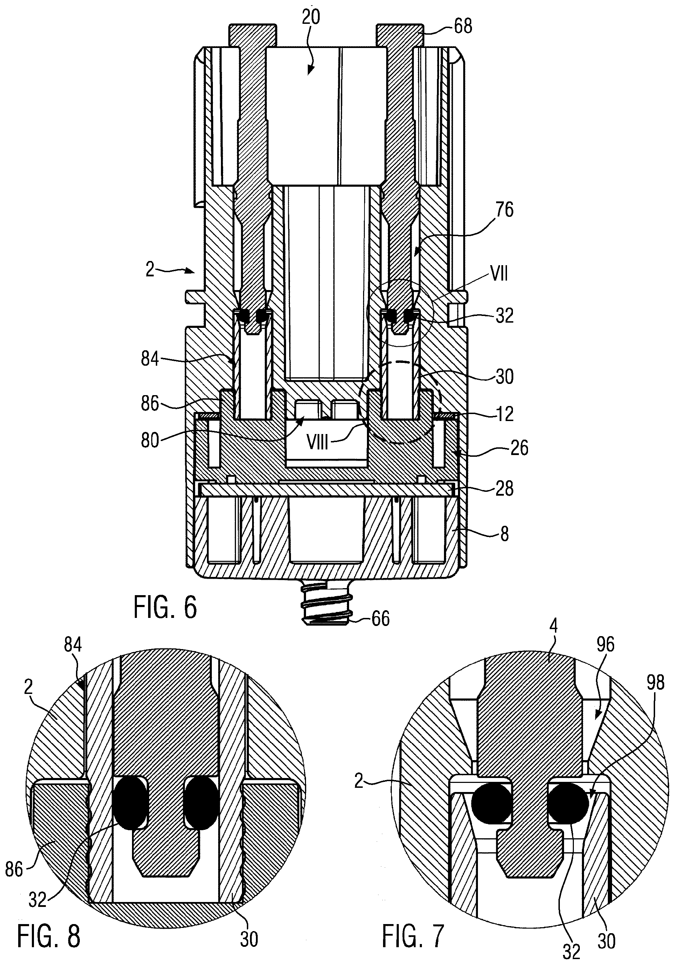

In axialer Verlängerung der Führungsbuchsen 76 bildet die Gehäusebasis 2 bis zu der Trennwand 80 reichende Zylindereinsatz-Aufnahmebohrungen 84 auf, die zur Aufnahme der Zylindereinsätze 30 angepasst ausgebildet sind und in etwa auf Höhe der Trennwand 80 radial verdickt sind, um zwischen dem Zylindereinsatz 30 und dem Material der Gehäusebasis 2 einen Ringraum auszubilden, in welcher ein vorspringender Ringkragen 86 des Ventilblocks 26 passt. Dieser Ringkragen 86 ist beispielsweise in den Fig. 6 und 9 dargestellt. Der Ringkragen 86 dient der dichtenden Verbindung zwischen dem Zylindereinsatz 30 und dem Ventilblock 26. Dabei ist - wie Fig. 8 verdeutlicht - eine konturierte Außenumfangsfläche der Zylindereinsätze 30 innerhalb des Ringkragens 86 aufgenommen und mit diesem auch formschlüssig verrastet. Jeder Zylindereinsatz 30 ist durch Pressen in den Ringkragen 86 eingesetzt und damit dichtend mit dem Ventilblock 26 verbunden.

Die Trennwand 80 bildet ferner eine sich zu dem Ventilblock 26 öffnende Ringnut aus, die zur Aufnahme RFID-Ringes 12 angepasst ausgebildet ist, sodass dieser RFID-Ring 12 zwischen der Trennwand 80 und dem Ventilblock 26 angeordnet werden kann (vgl. Fig. 5). Dabei zeigt Fig. 5 im unteren Teil dieser Ringnut eine Verdickung des RFID-Ringes 12, welcher den Datenträger darstellt. Der übrige, in radialer Richtung verschlankte Bereich des RFID-Ringes 12 dient der angemessenen Positionierung innerhalb der Gehäusebasis 2 (vgl. Fig. 1) und zudem als Spule zur Signalverstärkung eines beispielsweise von einem Handstück abgesetzten Signals, mit dem der Typ der in dem Handstück verbauten Düsengeometrie angezeigt wird.

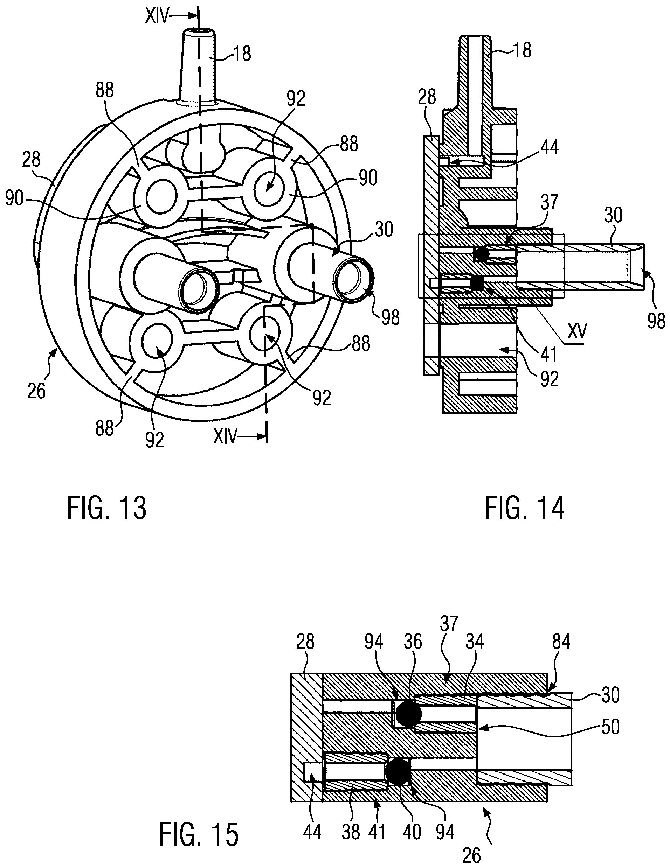

Wie die Fig. 5 und 13 verdeutlichen, ist auch der Ventilblock 26 als Bauteil gleicher Wandstärken ausgebildet und daher gut mittels Kunststoff-Spritzgießen herzustellen. Insbesondere Fig. 5 verdeutlicht mehrere sich in Bewegungsrichtung der Stößelkörper 4 erstreckende Stützrippen 88, die sich an der Trennwand 80 abstützen und Hülsensegmente 90 anbinden, die Durchtrittsbohrungen 92 für die Spannschrauben 6 ausformen, die mit den Durchtrittsbohrungen 82 durch die Trennwand 80 fluchten, wobei die Hülsensegmente 90 die zuvor erwähnten Ringflächen 62 zur Anlage des Abdeckelementes 28 ausbilden.

Die Fig. 14 und 15 verdeutlichen die Anordnungen der Ein- und Auslassventile 37, 41 in dem Ventilblock 26. Dieser Ventilblock 26 hat zur Aufnahme der entsprechenden Ventilbuchsen 34 und 38 angepasste Bohrungen 42, 50, denen jeweils in Strömungsrichtung des Fluids ein Aufnahmeraum 94 nachgelagert zugeordnet ist, in dem sich die Ventilkugel 36 bzw. 40 befindet. Diese Ventilkugel 36 bzw. 40 wirkt im geschlossenen Zustand des Ventils mit einer Ventilöffnung zusammen, die durch das strömungsferne Ende der entsprechenden Ventilbuchse 34, 38 gebildet ist. In Fig. 15 ist diese Position für die Ventilkugel 36 des Auslassventiles 37 gezeigt, wohingegen die Ventilkugel 40 des Einlassventils 41 die entsprechende Ventilöffnung freigibt. So verdeutlicht Fig. 15 einen Zustand, bei dem der Stößelkörper 4 den Hubraum innerhalb des Zylindereinsatzes 30 vergrößert und zu pumpende Flüssigkeit durch den Einlasskanal 44 in den Hubraum eingeleitet wird, wohingegen der Auslasskanal durch das Auslassventil 37 verschlossen ist. Die jeweiligen Ventilkugeln 36, 40 sind bei dem gezeigten Ausführungsbeispiel frei beweglich in dem Aufnahmeraum 34 vorgesehen und allein aufgrund der Durchmesserverhältnisse zwischen der Ventilöffnung und dem strömungsfernen Durchmesser des von der Ventilöffnung abgehenden und in dem Ventilblock 26 ausgebildeten Kanals in dem Ventilblock 26 unverlierbar gehalten. Zur Montage wird zunächst die entsprechende Kugel 36, 40 in den Aufnahmeraum 94 eingesetzt. Danach wird die Ventilbuchse 34 bzw. 38 in den Ventilblock 26 eingepresst. Danach sind die Ventile 37, 41 unverlierbar in dem Ventilblock 26 vormontiert.

Wie Fig. 15 ferner erkennen lässt, liegt der in den Ventilblock 26 eingepresste Zylindereinsatz 30 stirnseitig gegen die Ventilbuchse 34 des Auslassventiles 37 an, wodurch das auf der Druckseite der Pumpe vorgesehene Ventil 37 zusätzlich lagegesichert und gegen unerwünschtes Herauspressen aus der Presspassung zu der Ventilbuchse 36 gehindert wird.

Insbesondere die Fig. 7 verdeutlicht einen ersten konischen Einzug 96, der durch die Gehäusebasis 2 ausgebildet ist und den Zylindereinsatz 30 in Richtung auf den Antriebsbereich 20 vorgelagert vorgesehen. Dieser erste konische Einzug 96 erleichtert das Einbringen des Stößelkörpers 4 mit seinem vorderen Ende, an dem sich das Dichtelement 32 befindet, in den durch den Zylindereinsatz 30 gebildeten Zylinder. Beim Einbringen des Stößelkörpers 4 wird das Dichtelement 32 konzentrisch zu dem Zylindereinsatz 30 angeordnet und in etwa auf dessen Innendurchmesser gebracht. Ein zweiter konischer Einzug 98 wird durch den Zylindereinsatz 30 selbst ausgebildet. Innerhalb dieses zweiten konischen Einzugs 98 befindet sich das Dichtelement 32 in der in den Fig. 6 und 7 verdeutlichten Parkposition. So ist das Dichtelement 32 mit radialem Abstand zu dem Zylindereinsatz 30 vorgesehen. Der sich dadurch bildende Radialspalt erlaubt einen Durchtritt von Flüssigkeit und/oder Gas für die Sterilisation bzw. Desinfektion des Ausführungsbeispiels nach der Montage sämtlicher Bauteile. Diese Parkposition wird durch ein Sicherungselement festgelegt, welches vorliegend durch eine einteilig an der Gehäusebasis 2 angeformte Rastklinke 100 ausgebildet ist. Diese Rastklinke 100 ist insbesondere den Fig. 10 bis 12 zu entnehmen. Die Rastklinke 100 ist durch Freischneiden des antriebsseitigen Endes der Führungsbuchse 76 ausgebildet. Die Rastklinke 100 hat einen Verriegelungsvorsprung 102, der in den Fig. 9 und 10 verdeutlicht ist und in der Parkposition in eine Verriegelungsnut 104 eingreift, die zwischen zwei einteilig an dem Stößelkörper 4 einteilig ausgebildete ringförmige Vorsprünge 106, 108 ausgebildet ist (vgl. Fig. 10). Dabei bildet der vordere ringförmige Vorsprung 108 eine sich nahezu streng radial erstreckende Flanke der Verriegelungsnut 104 aus, wohingegen der hintere ringförmige Vorsprung 106 eine schräge Flanke hat, die das Vorschieben des Stößelkörpers 4 von der Parkposition in eine Pump- bzw. Betriebsposition, erleichtert. In einer Pump- bzw. Betriebsposition befindet sich das Dichtelement 32 in dichtender Anlage an der Innenumfangsfläche des Zylinders, vorliegend des Zylinderelementes 30. Dabei kann davon ausgegangen werden, dass die Fig. 9 und 10 die oberste Pumpposition und Fig. 8 die unterste Pumpposition wiedergeben. Zwischen diesen beiden Positionen nach den Fig. 8 und 9 findet der Hub des Stößelkörpers 4 statt.

Durch die Ausgestaltung von Rastklinke 100 und Verriegelungsnut 104 wird die zuvor beschriebene Parkposition gesichert. Axialer Druck gegen den Stößelkörper 4 von der Antriebsseite führt jenseits eines kritischen Wertes der Druckkraft dazu, dass die Parkposition aufgehoben und der Stößelkörper 4 tiefer in das Gehäuse und in die Pumpposition verschoben wird. In dieser Pumpposition führen die Vorsprünge 106, 108 den Stößelkörper 4 auch gegenüber der Führungsbuchse 76, die durch die Gehäusebasis 2 ausgebildet wird (vgl. Fig. 9, 10), wodurch eine höhere Laufruhe des Stößelkörpers 4 beim Pumpbetrieb erreicht wird. Insbesondere wird ein Knicken des Stößelkörpers 4 bei axialer Beanspruchung verhindert, sodass der Stößelkörper 4 aus einem relativ weichen Material, wie beispielsweise Kunststoff, hergestellt werden kann.

Wie Fig. 6 verdeutlicht, überragt der Stößelkörper 4 in der Parkposition mit seinem Hammerkopf 68 die Gehäusebasis 2, wodurch ein optischer Indikator zur Überprüfung der Parkposition gegeben ist. Nach dem Verbinden mit dem Antrieb, bei welchem zwangsläufig die Stößelkörper 4 von der Parkposition in eine Pumpposition überführt werden, liegen die antriebsseitigen Enden mit dem Hammerkopf 68 jeweils innerhalb der Gehäusebasis 2 und der durch die dort im Antriebsbereich 20 gebildete axial offene hintere Ausnehmung frei.

Wie die Beschreibung des Ausführungsbeispiels verdeutlicht, sind bei dem erfindungsgemäßen Pumpmodul zwischen dem Zylindereinsatz 30 und dem Dichtelement 32 die Ein- und Auslasskanäle 44, 64 ausgebildet. Diese erstrecken sich innerhalb einer Phasengrenze zwischen dem Ventilblock 26 und dem Abdeckelement 28. Der hier vorgesehene Einlasskanal 44 verteilt eingeleitete Flüssigkeit von einem oberen Ende nahe des Einlassanschlussstutzens 18 zu den jeweiligen Einlassventilen 41. Das Fluid wird in der Phasengrenze bis zu den Einlassventilen 41 am äußeren Rand der Phasengrenze geführt und umgibt dementsprechend zumindest teilweise den Auslasskanal 64. Dieser Auslasskanal 64 kommuniziert mit mehreren, vorliegend zwei, Auslassventilen 37. Innerhalb der Phasengrenze zwischen dem Abdeckelement 28 und dem Ventilblock 26 leitet der Auslasskanal 64 das unter Druck befindliche Fluid bis hin zu einem Sammelpunkt, der mit dem durch die Auslassanschlusstülle 66 gebildeten Abgabekanal fluchtet. Der Sammelpunkt befindet sich dabei ebenfalls innerhalb der Phasengrenze zwischen dem Abdeckelement 28 und dem Ventilblock 26. Insbesondere wird der überwiegende Teil von Einlasskanal 44und/oder Auslasskanal 64 innerhalb der Phasengrenze zwischen dem Ventilblock 26 und dem Abdeckelement 28 gebildet. Der überwiegende Teil stellt dabei zumindest 50 %, bevorzugt 60 % der gesamten Länge des Strömungsweges des entsprechenden Kanals innerhalb des Pumpmodules dar. Dieser Strömungsweg beginnt für die Einlassseite mit der Einlassöffnung des Einlassstutzens 18 und endet an dem Einlassventil 41. Der entsprechende Weg beginnt auslassseitig mit der durch die Auslassanschlusstülle 66 gebildete Öffnung und endet an dem Auslassventil 37, vorliegend dem Aufnahmeraum 94 des entsprechenden Ventils 37.

Bedeutsam für die Erfindung ist ferner die Pumpeinheit 10, die aus dem Ventilblock 26 und dem Abdeckelement 28 mit den darin eingebauten Ventilen 37, 41 und den Zylindereinsätzen 30 besteht. Diese Pumpeinheit 10 ist vormontiert. Dabei kann die Erfindung auch insofern variiert werden, indem der Zylinder durch die Gehäusebasis 2 selbst oder ein in die Gehäusebasis 2 aufgenommenes Zylinderelement gebildet wird, welches dichtend gegen den Ventilblock 26 angelegt wird. So ist vorstellbar, dass der in Fig. 7 zu erkennende Kragen im Anschluss an den ersten konischen Einzug 96 unmittelbar an einem Zylindereinsatz anliegt und diesen durch Vorspannung der Gehäusebasis 2 unter Vorspannung gegen den Ventilblock 26 andrückt, und zwar zusammen mit einem O-Ring, der an der Phasengrenze zwischen der Gehäusebasis 2 und dem Ventilblock 26 angeordnet werden kann und den so vorgesehenen Zylindereinsatz abdichtet.

Weiterhin ist bedeutsam, dass eine Parkposition definiert ist, in welcher der durch die Stößelkörper 4 gebildete Pumpkolben festgelegt ist, derart, dass bei gewissem axialem Druck der Stößelkörper 4 aus der Parkposition heraus in eine Pumpposition verschoben wird. In der Parkposition liegt jedenfalls das Dichtelement 32 nicht an der Innenumfangsfläche des zugeordneten Pumpzylinders an. Das Dichtelement 32 ist regelmäßig mit radialem Abstand zu benachbarten Gehäuseteilen des Pumpmodules vorgesehen, sodass die Sterilisation bzw. Desinfektion an dem Zylinder und dem Kolben vorbei erfolgen kann. Sämtliche strömungsführenden Teile des Pumpmodules werden hierbei vollständig mit dem Desinfektions- bzw. Sterilisationsmittel überstrichen und damit wirkungsvoll sterilisiert.

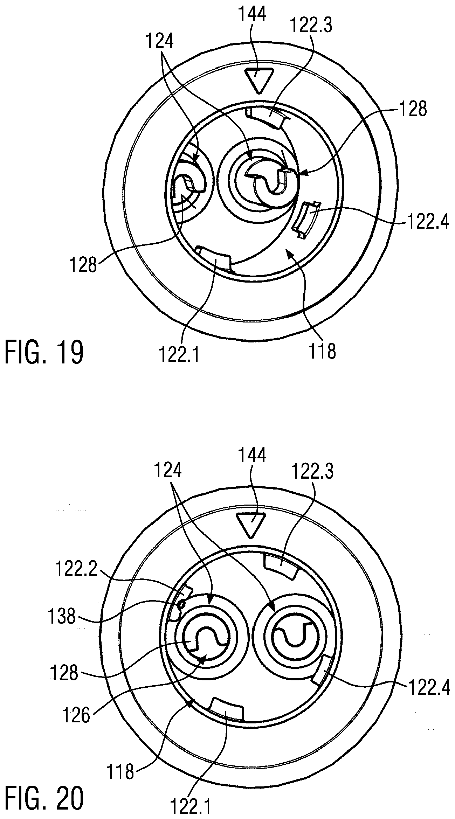

Die Figur 19 zeigt eine perspektivische Seitenansicht eines Ausführungsbeispiels einer Antriebseinheit 110 mit einem in einem Antriebsgehäuse 112 vorgesehenen Antrieb, bei dem es sich um einen elektrischen Antrieb handelt. Von dem Antriebsgehäuse 112 ragt eine Halterung 114 zur Halterung eines Flüssigkeitsbeutels ab. An dem Antriebsgehäuse 112 liegen ferner verschiedene Bedienelemente 116 frei, die der Steuerung des Antriebs sowie dem Ein- bzw. Ausschalten des Antriebs dienen. Bezugszeichen 118 kennzeichnet eine im Wesentlichen zylindrische Ausnehmung, in die ein mit Bezugszeichen 120 gekennzeichnetes Pumpmodul nach den Figuren 1 bis 17 eingesetzt ist, welches gegenüber diesen Figuren vereinfacht dargestellt ist. Die Gehäusebasis 2 hat nach innen in die Ausnehmung 118 vorspringende Nocken 122, die Ausführungsbeispiele für Formschlusselemente der vorliegenden Erfindung sind. Es sind vorliegend vier Nocken 122 auf dem Umfang verteilt vorgesehen. Die mit Bezugszeichen 122.4 gekennzeichnete Nocke kann eine geringere radiale Erstreckung und eine geringere Erstreckung in Umfangsrichtung als die übrigen Nocken 122.1 bis 122.3 haben, um eine eindeutige Zuordnung des Pumpmoduls 120 zu erlauben. Andere Arten einer Poka-yoke-Gestaltung sind denkbar. So können Nuten mit unterschiedlichem Winkelversatz relativ zueinander auf der Außenumfangsfläche des Gehäuses, namentlich der Gehäusebasis 2, vorgesehen sein, so dass das Pumpmodul 120 lediglich in einer vorbestimmten Weise in die Ausnehmung 118 eingesetzt werden kann. In der Ausnehmung 118 liegen ferner Antriebselemente in Form von Antriebsstößeln 124 frei, die mit dem innerhalb des Antriebsgehäuses 112 vorgesehenen Antrieb verbunden und in Längsrichtung reversierend angetrieben hin und her beweglich sind. Die Antriebsstößel 124 bilden eine Anschlagfläche 126 aus. Vorliegend sind zwei Antriebsstößel 124 vorgesehen. Die Anschlagfläche 126 wird durch eine in der Draufsicht C-förmige Klaue 128 überragt, die zwischen sich und der Anschlagfläche 126 eine Hammerkopfaufnahme 130 ausbildet.

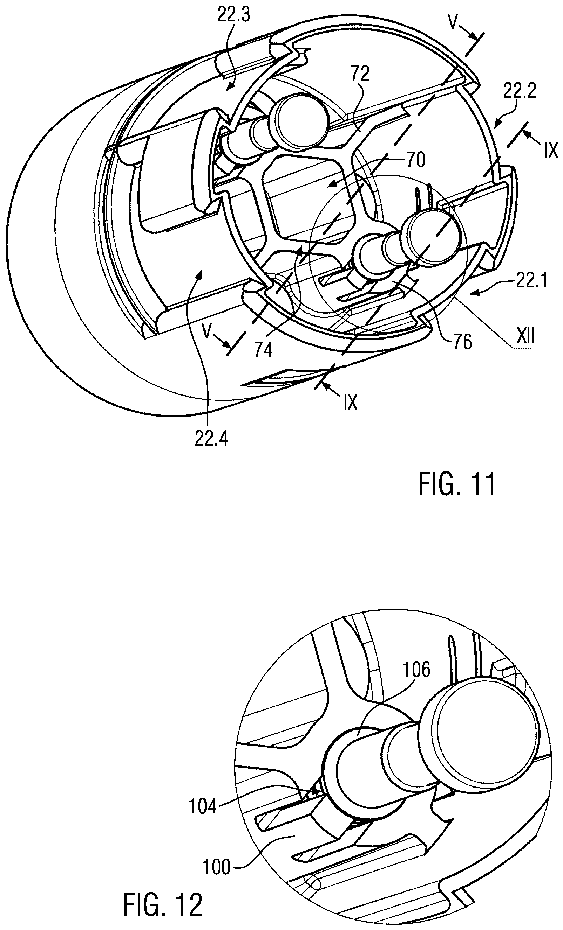

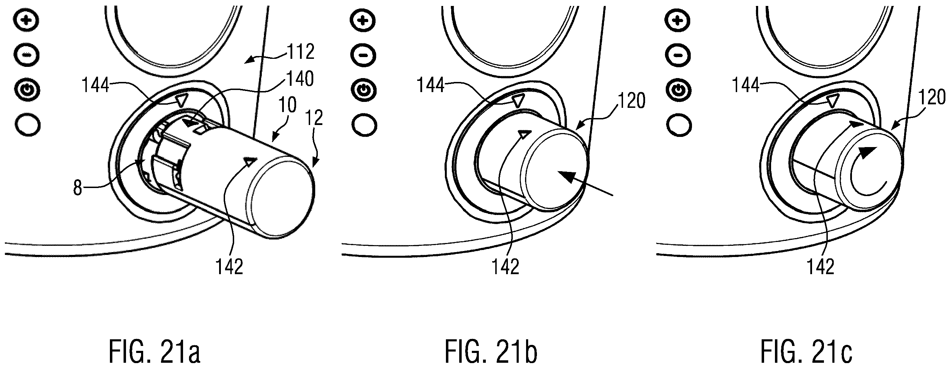

Wie insbesondere die Figuren 11 und 16 erkennen lassen, ist von den vier Nuten 22 am Außenumfang der Gehäusebasis 2, die sich streng in axialer Richtung entlang der Mittellängsachse L erstrecken, die mit Bezugszeichen 22.4 gekennzeichnete Nut zur exakten Aufnahme der kleineren Nocke 122.4 angepasst ausgebildet. Durch das Zusammenwirken insbesondere der kleineren Nocke 122.4 mit der kleineren Nut 22.4 wird eine eineindeutige Ausrichtung des Pumpmoduls 120 beim Fügen, d.h. beim Einschieben des Pumpmoduls 120 in die Ausnehmung 116 vorgegeben. Damit kann das Pumpmodul 120 nur in einem Winkel rechtwinklig zu einer in Figur 21c gezeigten Endlage um 30° versetzt eingeführt werden. Diese geschwenkte Lage ist in Figur 21b verdeutlicht. Der Hammerkopf 68 überragt einen endseitigen Pumpkolbenabschnitt 132 jedes einzelnen Pumpkolbens 4, der einen geringeren Durchmesser als der übrige Pumpkolben 4 hat. Der Hammerkopf 68 definiert das stirnseitige, anschlussseitige Ende des Pumpkolbens 4 und bildet hier eine Gegenfläche 134 zu der Anschlagfläche 126 aus.

Die Nut 22 bildet zusammen mit der Quernut 24 eine Führung eines Bajonettverschlusses mit dem jeweiligen Nocken 122, um zunächst eine axiale Einführbewegung zu führen, die ihr Ende dann findet, wenn die Nocken 122 gegen das innenseitige untere Ende der Nuten 22 anstoßen, um danach in einer Schwenkbewegung in die Quernut 24 schwenkt und damit axial arretiert zu werden. In der endseitig gegen die Quernut 24 anliegenden Endlage kann ein Rastvorsprung wirksam sein, der eine Verdrehsicherung zwischen dem Pumpmodul 112 und dem Antriebsgehäuse 2 ausbildet, sodass das Pumpmodul 112 in seiner Endlage gesichert ist.

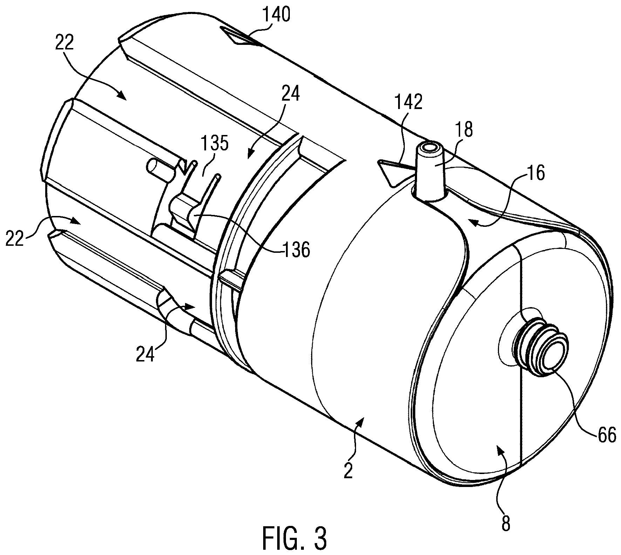

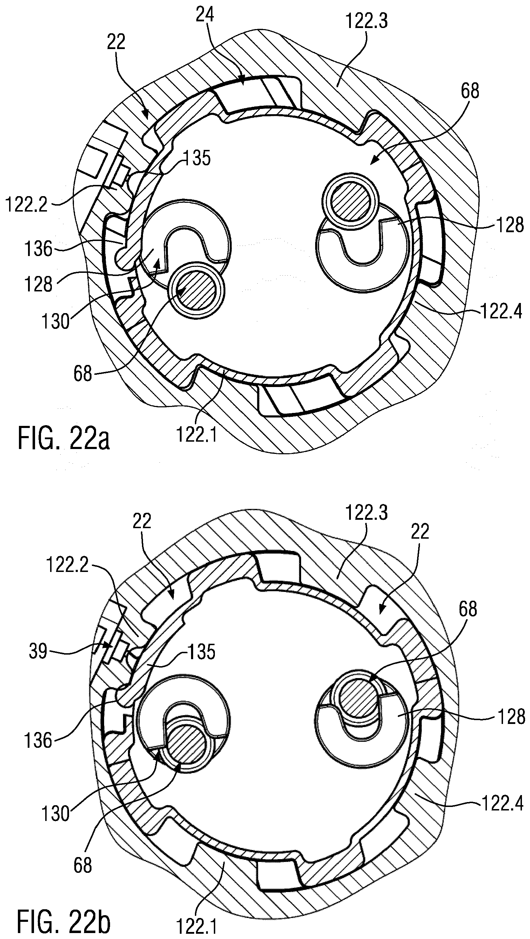

In Fig. 22a ist ferner innerhalb der Quernut 24 ein an einem Federarm 135 ausgebildeter Rast- und Schaltvorsprung 136 eingezeichnet, der in der Quernut 24 freiliegt und fest an der Gehäusebasis 2 ausgebildet ist (vgl. Fig. 3). Diesem Rast- und Schaltvorsprung 136 ist ein mittig in der Nocke 122.2 vorgesehener Schalter 138 zugeordnet. Der Schalter 138 ist in radialer Richtung nach innen in Bezug auf die Ausnehmung 118 vorgespannt und wirkt dementsprechend mit dem Rast- und Schaltvorsprung 136 zusammen. Durch Betätigung dieses Schalters 138 durch den Rast- und Schaltvorsprung 136 wird erst die Möglichkeit gegeben, die Antriebsstößel 124 anzutreiben. Ist dementsprechend das Pumpmodul 10 nicht in der vorgeschriebenen Weise mit der Antriebseinheit 1 verbunden, kann die Antriebseinheit nicht betrieben werden. Zusätzlich ist das Antriebsgehäuse 112 mit einer Leseeinheit versehen, welche die richtige Ausrichtung des RFID-Ringes 12 und damit des Pumpmoduls 120 relativ zu dem Antriebsgehäuse 112 erkennt und erst dann den Abtrieb freigibt. Damit wird ein Betrieb der Vorrichtung bei überbrücktem Schalter 138 verhindert.

Die Figuren 21a bis c zeigen das Einführen des Pumpmoduls 120 in die Ausnehmung 118. Wie bereits zuvor erwähnt, wird das Pumpmodul 120 um 30° im Gegenuhrzeigersinn relativ zu der Endlage zunächst geschwenkt, um die Nocken 122 mit den Nuten 22 zur Überdeckung zu bringen (vgl. Figur 21a). Die geschwenkte Position wird durch einen Ausrichtungspfeil 140 gekennzeichnet, der in Figur 3 deutlich zu erkennen ist und in Figur 21a mit einer gehäuseseitig vorgesehenen Gegenmarkierung 144 fluchtet. In dieser relativen Ausrichtung kann das Pumpmodul 120 nunmehr in die Ausnehmung 118 eingeschoben werden. Diese axiale Einschiebebewegung wird geführt durch die Nocken 122, die in die korrespondierend hierzu ausgebildeten Nuten 22 eingreifen. In der Darstellung nach Figur 21b ist dieses axiale Einschieben, das in Figur 21b mit einem gradlinigen Pfeil verdeutlicht wird, beendet. Das Pumpmodul 120 ist nunmehr maximal in die Ausnehmung 118 eingeschoben. Danach wird das Pumpmodul 120 um 30° im Uhrzeigersinn geschwenkt, wie dies der Pfeil in Figur 21c andeutet. Nach dieser Schwenkbewegung um 30° hat das Pumpmodul 120 seine Endposition erreicht. Die Endposition wird dem Benutzer auch durch einen auf dem Außenumfang der Gehäusebasis 2 vorgesehenen Richtungspfeil 142 angeben, der in der Endposition mit der Gegenmarkierung 144, die an dem Antriebsgehäuse 2 vorgesehen ist, fluchtet. Der Richtungspfeil 142 gibt auch die Richtung des Einführens für das Pumpmodul 120 in die Ausnehmung 118 vor.

Beim Fügen von Pumpmodul 120 und Antriebsgehäuse 112 werden die Antriebsstößel 124 und die Pumpkolben 4 einander angenähert. Aufgrund der axialen Führung der Nocken 122 in den Nuten 22 befindet sich die durch den Hammerkopf 68 gebildete Gegenfläche 134 zumindest teilweise über der durch den Antriebsstößel 124 gebildeten Anschlagfläche 126 (vgl. Figur 22a). So führt eine fortschreitende axiale Bewegung schließlich dazu, dass der Pumpkolben 4 endseitig gegen die Anschlagfläche 126 angelegt wird. Mit weiter zunehmender Annäherung des Pumpmoduls 120 an das Antriebsgehäuse 112 wird die Parkposition aufgehoben und der Pumpkolben 4 tiefer in die Gehäusebasis 2 und in eine Pumpposition gedrängt. Es ergibt sich danach keine weitere relative axiale Bewegung zwischen dem Antriebsstößel 124 und dem zugeordneten Pumpkolben 4.

Der jeweilige Hammerkopf 68 der beiden Pumpkolben 4 befindet sich dabei in einer exzentrischen Position relativ zu dem Mittelpunkt des Antriebsstößels 124, die in Figur 22a gezeigt ist. Üblicherweise wird nach dem axialen Anlegen beider Pumpkolben 4 an die Antriebsstößel 124 die Gehäusebasis 2 um einen weiteren, geringfügigen Weg axial relativ zu dem Antriebsgehäuse 2 verschoben, sodass sichergestellt wird, dass bis zum Erreichen der axialen Endlage beim Fügen von Pumpmodul 120 und Antriebsgehäuse 112 jederzeit zuverlässig eine axiale Anlage des Pumpenkolbens 4 an dem Antriebsstößel 124 erreicht wird, bevor die Gehäusebasis 2 relativ zu dem Antriebsgehäuse 112 geschwenkt wird. Jedenfalls sollte die Ausgestaltung so sein, dass bei jeder erdenklichen Positionierung des Antriebsstößels 124, selbst bei einer Positionierung des Antriebsstößels 124 in der tiefsten Lage innerhalb der Ausnehmung 8 eine sichere Anlage des Pumpkolbens 4 gegen den Antriebsstößel 124 nach Beendigung der axialen Einschiebebewegung erreicht ist.

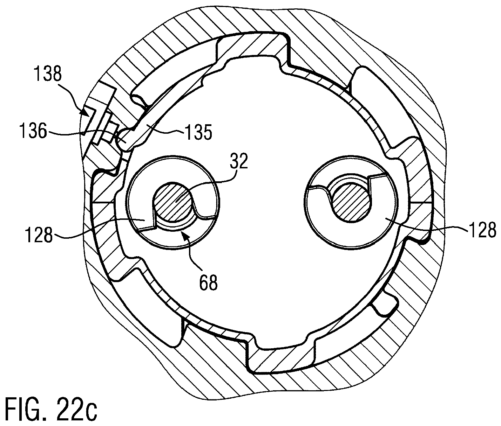

Nach Erreichen dieser axialen Endlage wird das Pumpmodul 120 nunmehr im Uhrzeigersinn geschwenkt. Dadurch werden - wie die Figuren 22a bis 22c verdeutlichen - die exzentrisch zu dem Mittelpunkt dieser Schwenkbewegung angeordneten Hammerköpfe 68 mit ihrer Gegenfläche 134 gleitend auf der Anschlagfläche 126 relativ zu dem Antriebsstößel 124 verschoben, und zwar in einer Ebene, die sich rechtwinklig zu der Einschieberichtung erstreckt. Die zuvor exzentrische Anordnung der Pumpkolben 4 relativ zu den Antriebsstößeln 124 gemäß Figur 22a nähert sich danach über eine in Figur 22b gezeigte Zwischenposition der in Figur 22c gezeigten Endposition an. In dieser Endposition stoßen die Nocken 122 gegen Anschläge an, die durch die Quernuten 24 gebildet sind. Die Gehäusebasis 2 wird üblicherweise gegen das Antriebsgehäuse 2 verriegelt. Die Pumpkolben 4 sind im Wesentlichen konzentrisch zu den Antriebsstößeln 124 angeordnet. Jede Klaue 128 übergreift den zugeordneten Hammerkopf 68. So ist der Hammerkopf 68 durch Übergriff der die Klaue 128 aufweisenden Hammerkopfaufnahme 130 axial formschlüssig gehalten. Üblicherweise ist die Hammerkopfaufnahme 130 in axialer Richtung exakt auf die Höhe des Hammerkopfes 68 abgestimmt, sodass sich eine spielfreie axiale formschlüssige Verbindung zwischen dem Antriebsstößel 124 und dem Pumpkolben 4 ergibt.

### Bezugszeichenliste

- 2: Gehäusebasis
- 4: Stößelkörper/Pumpkolben
- 6: Spannschraube
- 8: Kopfelement
- 10: Pumpeinheit
- 12: RFID-Ring
- 14: Ausgabebereich
- 16: Axialschlitz
- 18: Einlassanschlussstutzen
- 20: Antriebsbereich
- 22: Nut
- 24: Quernut
- 26: Ventilblock
- 28: Abdeckelement
- 30: Zylindereinsatz
- 32: Dichtelement
- 34: Ventilbuchse
- 36: Ventilkugel
- 37: Auslassventil
- 38: Ventilbuchse
- 40: Ventilkugel
- 41: Einlassventil
- 42: Einlassventilbohrung
- 44: Einlasskanal
- 46: Vorsprung
- 50: Auslassventilbohrung
- 52: Passelement
- 54: Dichtfläche
- 56: Passbohrung
- 58: Auslassbohrung
- 60: Durchgangsbohrungen
- 62: Ringfläche
- 64: Auslasskanal
- 66: Auslassanschlusstülle
- 68: Hammerkopf
- 70: mittlere Ausnehmung
- 72: radialer Steg
- 74: Polygonstruktur
- 76: Führungsbuchse
- 78: weiterer radialer Steg
- 80: Trennwand
- 82: Durchtrittsbohrung
- 83: Dichtring
- 84: Zylindereinsatz-Aufnahmebohrung
- 86: Ringkragen
- 88: Stützrippe
- 90: Hülsensegment
- 92: Durchtrittsbohrung
- 94: Aufnahmeraum
- 96: erster konischer Einzug
- 98: zweiter konischer Einzug
- 100: Rastklinke
- 102: Verriegelungsvorsprung
- 104: Verriegelungsnut
- 106: ringförmiger Vorsprung
- 108: ringförmiger Vorsprung
- 110: Antriebseinheit
- 112: Antriebsgehäuse
- 114: Halterung
- 116: Bedienelement
- 118: Ausnehmung
- 120: Pumpmodul
- 122: Nocken
- 124: Antriebsstößel
- 126: Anschlagfläche
- 128: Klaue
- 130: Hammerkopfaufnahme
- 132: Pumpkolbenabschnitt
- 134: Gegenfläche
- 135: Federarm
- 136: Rast- und Schaltvorsprung
- 138: Schalter
- 140: Ausrichtungspfeil
- 142: Richtungspfeil
- 144: Gegenmarkierung
- L: Mittellängsachse

## Patentansprüche

1. Pumpmodul mit einem Gehäuse (2, 8, 26, 28), in dem zumindest ein Pumpkolben (4) reversierend beweglich gelagert ist, der mit zumindest einem Dichtelement (32) versehen ist, das in einem Pumpbetrieb dichtend an einem Zylinder (30) anliegt, wobei der Pumpkolben (4) in einer Parkposition, in der das Dichtelement (32) nicht an dem Zylinder (30) anliegt, festlegbar ist,
**gekennzeichnet durch** ein an dem Gehäuse (2, 8, 26, 28) angeordnetes Sicherungselement (100), das in der Parkposition mit einem an dem Pumpkolben (4) vorgesehenen Sicherungsgegenelement (104, 106, 108) zur Festlegung des Pumpkolbens (4) in der Parkposition zusammenwirkt.

2. Pumpmodul nach Anspruch 1, **dadurch gekennzeichnet, dass** das Sicherungselement bzw. das Sicherungsgegenelement zwei mit axialem Abstand zueinander vorgesehene Radialvorsprünge (106, 108) umfasst, zwischen denen das andere von Sicherungselement (100) bzw. das Sicherungsgegenelement in der Parkposition eingreift.

3. Pumpmodul nach Anspruch 1 oder 2, **dadurch gekennzeichnet, dass** das Sicherungsgegenelement (106, 108) im Pumpbetrieb in einem Führungszylinder (76) des Gehäuses (2) geführt ist, der dem Zylinder axial vorgelagert ist.

4. Pumpmodul nach einem der vorherigen Ansprüche, **dadurch gekennzeichnet, dass** dem Zylinder ein konischer Einzug (96, 98) vorgelagert ist, dessen kleinster Innendurchmesser im Wesentlichen dem Innendurchmesser des Zylinders entspricht.

5. Pumpmodul nach Anspruch 4, **dadurch gekennzeichnet, dass** das Dichtelement in der Parkposition in dem konischer Einzug (96, 98) angeordnet ist.

6. Pumpmodul nach einem der vorherigen Ansprüche, **dadurch gekennzeichnet, dass** der Pumpkolben einen aus einem Kunststoff gebildeten Stößelkörper (64) aufweist, der an einem Ende ein Formschlusselement (68) zur Koppelung des Kolben (4, 32) mit einem dem Kolben (4, 32) zugeordneten Antrieb aufweist und an seinem anderen Ende zur Befestigung des Dichtelementes (32) angepasst ausgebildet ist.

7. Pumpmodul nach einem der vorherigen Ansprüche, **dadurch gekennzeichnet, dass** das eine Ende in der Parkposition von dem Gehäuse (2, 8, 26, 28) überragt und/oder von einer auf das Gehäuse gesetzten Abdeckkappe überdeckt ist.

8. Pumpmodul nach einem der vorherigen Ansprüche, **dadurch gekennzeichnet, dass** in der Parkposition zwischen dem Dichtelement und einer das Dichtelement umfänglich umgebenden Gehäusewandung ein Spalt von zumindest 1/10 mm, bevorzugt zumindest 2/10 mm und besonders bevorzugt zumindest 3/10 mm eingestellt ist.

9. Pumpmodul nach einem der vorherigen Ansprüche, **gekennzeichnet durch,** einen dem Pumpkolben (4, 32) zugeordneten Zylindereinsatz (30), der in einem Pumpbetrieb zwischen sich und dem Stößelkörper (64) das Dichtelement (6) einschließt.

10. Pumpmodul nach Anspruch 9, **dadurch gekennzeichnet, dass** der Zylindereinsatz (30) stirnseitig gegen einen Ventilblock (10) anliegt, der Ein- und Auslasskanäle (23, 24) zu dem Zylindereinsatz (30) ausbildet und Ein- und Auslassventilelemente (18, 20) in sich aufnimmt.

## Claims

1. Pump module comprising a casing (2, 8, 26, 28) in which at least one pump piston (4) is mounted in a reciprocating manner and provided with at least one sealing element (32) which in a pumping operation sealingly abuts against a cylinder (30), wherein said pump piston (4) can be fixated in a parking position in which said sealing element (32) does not abut against said cylinder (30), **characterized by** a locking element (100) which is arranged on said casing (2, 8, 26, 28) and which in the parking position interacts with a locking counter-element (104, 106, 108) provided on said pump piston (4).

2. Pump module according to claim 1, **characterized in that** said locking element or said locking counter-element comprises two radial projections (106, 108) provided at an axial distance from one another between which the other of said locking element (100) or said locking counter-element engages in the parking position.

3. Pump module according to claim 1 or 2, **characterized in that** said locking counter-element (106, 108) is in the pumping operation guided in a guide cylinder (76) of said casing (2) being axially disposed ahead of said cylinder (30).

4. Pump module according to one of the preceding claims, **characterized in that** a conical in-feed device (96, 98) is disposed ahead of said cylinder (30), the smallest inner diameter of which substantially corresponds to the inner diameter of said cylinder (30).

5. Pump module according to claim 4, **characterized in that** said sealing element is in the parking position arranged in said conical in-feed device (96, 98).

6. Pump module according to one of the preceding claims, **characterized in that** said pump piston comprises a plunger body (64) formed from plastic material and at one end comprising a positive-fit element (68) for coupling said piston (4, 32) to a drive associated with said piston (4, 32) and at its other end being formed adapted for fastening said sealing element (32).

7. Pump module according to one of the preceding claims, **characterized in that** the said casing (2, 8, 26, 28) in the parking position projects over said one end and/or the latter is covered by a cover cap placed on said casing.

8. Pump module according to one of the preceding claims, **characterized in that** a gap of at least 1/10 mm, preferably at least 2/10 mm and particularly preferably at least 3/10 mm is in the parking position set between said sealing element and a casing wall circumferentially surrounding said sealing element.

9. Pump module according to one of the preceding claims, **characterized by** a cylinder insert (30) which is associated with said pump piston (4, 32) and which in pumping operation encloses said sealing element (6) between itself and said plunger body (64).

10. Pump module according to claim 9, **characterized in that** said cylinder insert (30) at the face side abuts against a valve block (10) which forms inlet and outlet passages (23, 24) to said cylinder insert (30) and receives inlet and outlet valve elements (18, 20) within itself.

## Revendications

1. Module de pompe comprenant un carter (2, 8, 26, 28) dans lequel est monté mobile, de façon réversible, au moins un piston de pompe (4), qui est pourvu d'au moins un élément d'étanchéité (32) s'appuyant de manière étanche contre un cylindre (30) au cours d'un fonctionnement en mode pompage, le piston de pompe (4) pouvant être fixé dans une position de repos, dans laquelle l'élément d'étanchéité (32) ne s'appuie pas contre le cylindre (30),
**caractérisé par** un élément d'arrêt de sécurisation (100) agencé sur le carter (2, 8, 26, 28) et qui, dans la position de repos, interagit avec un élément d'arrêt de sécurisation conjugué (104, 106, 108) prévu sur le piston de pompe (4), en vue de fixer le piston de pompe (4) dans la position de repos.

2. Module de pompe selon la revendication 1, **caractérisé en ce que** l'élément d'arrêt de sécurisation, ou respectivement l'élément d'arrêt de sécurisation conjugué, comporte deux protubérances radiales (106, 108) prévues à distance axiale l'une de l'autre, et entre lesquelles s'engage, dans la position de repos, l'autre desdits élément d'arrêt de sécurisation (100) et respectivement élément d'arrêt de sécurisation conjugué.

3. Module de pompe selon la revendication 1 ou la revendication 2, **caractérisé en ce que** lors du fonctionnement en mode pompage, l'élément d'arrêt de sécurisation conjugué (106, 108) est guidé dans un cylindre de guidage (76) du carter (2), qui est monté axialement en amont du cylindre.

4. Module de pompe selon l'une des revendications précédentes, **caractérisé en ce que** le cylindre est précédé d'une entrée conique (96, 98) dont le diamètre intérieur le plus petit correspond sensiblement au diamètre intérieur du cylindre.

5. Module de pompe selon la revendication 4, **caractérisé en ce que** dans la position de repos, l'élément d'étanchéité est agencé dans l'entrée conique (96, 98).

6. Module de pompe selon l'une des revendications précédentes, **caractérisé en ce que** le piston de pompe comprend un corps de poussoir (64) réalisé en une matière plastique, qui, à une extrémité, présente un élément de liaison par complémentarité de formes (68) destiné au couplage du piston (4, 32) à un entraînement associé au piston (4, 32), et à sont autre extrémité, est réalisé de manière à être adapté à la fixation de l'élément d'étanchéité (32).

7. Module de pompe selon l'une des revendications précédentes, **caractérisé en ce que** dans la position de repos, ladite une extrémité dépasse du carter (2, 8, 26, 28) et/ou est recouverte par un capuchon de recouvrement monté sur le carter.

8. Module de pompe selon l'une des revendications précédentes, **caractérisé en ce que** dans la position de repos, il est réglé, entre l'élément d'étanchéité et une paroi de carter entourant de manière périphérique l'élément d'étanchéité, un interstice d'une valeur d'au moins 1/10 mm, de préférence d'au moins 2/10 mm et de manière particulièrement préférée d'au moins 3/10 mm.

9. Module de pompe selon l'une des revendications précédentes, **caractérisé par** un insert de cylindre (30), qui est associé au piston de pompage (4, 32), et qui, au cours du fonctionnement en mode pompage, enserre entre lui-même et le corps de poussoir (64), l'élément d'étanchéité (6).

10. Module de pompe selon la revendication 9, **caractérisé en ce que** l'insert de cylindre (30) s'appuie frontalement contre un bloc de valve (10), qui forme des canaux d'entrée et de sortie (23, 24) vers l'insert de cylindre (30), et loge des éléments de valve d'entrée et de sortie (18, 20).
